# EUROPEAN PATENT SPECIFICATION

(11) **EP 3 260 155 B1**
(45) Date of publication and mention of the grant of the patent: **02.01.2019**
(21) Application number: 17157148.2
(22) Date of filing: 21.02.2017
(51) Int. Cl.: A61M 25/00

(54) **CATHETER**
KATHETER
CATHÉTER

(30) Priority: 24.06.2016 JP 2016125660
(43) Date of publication of application: 27.12.2017
(73) Proprietor: ASAHI INTECC CO., LTD., Nagoya-shi, Aichi 463-0024 (JP)
(72) Inventor: NAKAYAMA, Noriyuki, Nagoya-shi, Aichi 463-0024 (JP); NIHONMATSU, Masaaki, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 2 695 635
- EP-A1- 2 762 187
- EP-A2- 2 923 724
- WO-A2-00/35527
- US-A1- 2001 051 790
- US-B2- 6 508 804

## Description

### Field

The present invention relates to a catheter that is inserted into a body lumen such as a blood vessel and a urinary duct.

### Background

The catheter inserted into a body lumen such as a blood vessel and an urinary duct generally includes an inner layer that is a long hollow tubular body, a reinforcing body that covers an outer periphery of the inner layer, and an outer layer that covers an outer periphery of the reinforcing body.

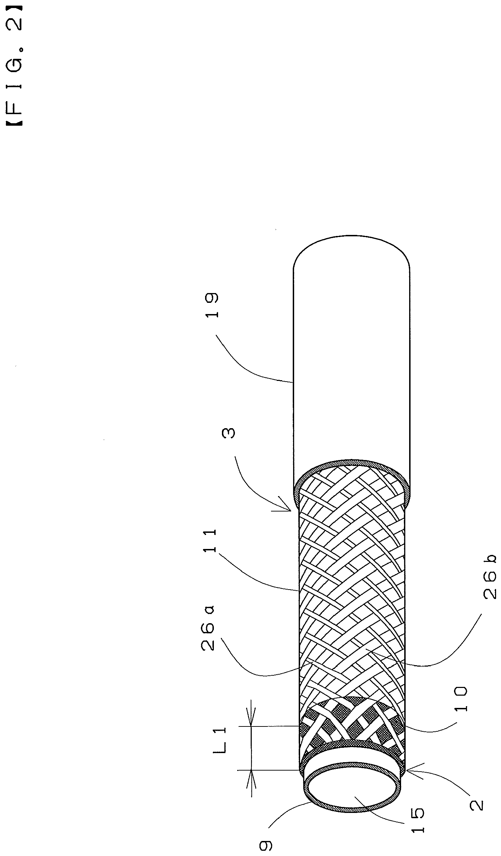

It is recognized that Cited Document 1, for example, describes that in the catheter (catheter) 10 including the inner layer (inner tubular member) 30, the reinforcing body (filament) 100 and the like covering the outer periphery of the inner layer 30, and the outer layer (outer layer) 190 covering the outer periphery of the reinforcing body 100, the reinforcing body 100 and the like are folded back on the outer periphery of the inner layer 30 to form double layers and triple layers (see FIG. 2 to FIG. 6, etc.).

In Cited Document 1, the end of the reinforcing body 100 and the like are held between the inner layer 30 and the radiopaque ring 70 (26th to 31st lines of 8th column). Here, the radiopaque ring 70 is used so that a user of the catheter can visually recognize a position of the catheter during procedures, and the radiopaque ring 70 functions as a so-called marker here.

In the catheter described in Cited Document 1, the reinforcing body is folded back on the outer periphery of the inner layer to form a plurality of layers, in the middle part of the catheter. However, in the case where the folded part is moved to an end of the reinforcing body, the reinforcing body is loosened. Therefore, in the case where the folded part of the reinforcing body is moved to an end of the reinforcing body, it is necessary to fix the end of the reinforcing body to the inner layer with a fixing tool such as a ring, as described in Cited Document 1.

Moreover, the catheter described in Cited Document 1 separately requires a separate radiopaque ring 70 as a marker so that a user of the catheter can visually recognize a position of the catheter during procedures.

### Citation List

### Patent Literature

Patent Literature 1: US Patent No. 6508804 Summary

### Technical Problem

In view of the above-described problems, the present invention aims at providing a catheter that does not require a fixing tool for fixing an end of a reinforcing body to an inner layer and/or a separate marker allowing a user of the catheter to visually recognize a position of the catheter during procedures.

### Solution to Problem

In order to achieve the above-described object, the first aspect of the present invention is a catheter comprising: an inner layer that is a long hollow tubular body, a tubular braid that covers an outer periphery of the inner layer, and a tubular outer layer that covers an outer periphery of the braid, wherein the braid is folded back at least at one of the longitudinal ends of the inner layer to form a multiple folded part so that a tip end of the braid is in contact with the inner layer and is arranged in the lowest layer of the folded part. The tip end of the braid refers to a distal end or a proximal end of the tubular braid in the unfolded state.
The tubular braid may cover the outer periphery of the inner layer from one longitudinal end to the other longitudinal end of the inner layer.

The second aspect of the present invention is the invention of the first aspect, in which the tip end of the braid is positioned in an area between the two longitudinal ends of the folded part.

The third aspect of the present invention is the invention of the first aspect or the second aspect, further including a distal end tip connected to a distal end of a catheter main body, and the inner layer and the braid extend to the distal end tip. The catheter main body includes the inner layer, the braid and the outer layer. The distal end tip may include the folded part of the braid.

The fourth aspect of the present invention is the invention of any one of the first aspect to the third aspect, in which the braid is formed of a metal material. Advantageous Effects of Invention

The catheter according to the first aspect of the present invention includes a catheter having an inner layer that is a long hollow tubular body, a tubular braid that covers an outer periphery of the inner layer , and an outer tubular layer covering an outer periphery of the braid, in which the braid is folded back along the outer periphery of the inner layer at least at one of the longitudinal ends of the inner layer to form a multiple folded part so that the tip end of the braid is in contact with the inner layer and is arranged in the lowest layer of the folded part. Thus, the braid can be fixed to the inner layer without requiring a fixing tool for fixing the end of the braid to the inner layer.

In the catheter according to the first aspect, the tip end of the braid is fixed in a series of operation by a so-called braider, which exerts the effect of facilitating fixing of the tip end of the braid.

The catheter according to the second aspect is the catheter according to the invention of the first aspect, in which the tip end of the braid is positioned in an area between the two longitudinal ends of the folded part. Thus, the tip end of the braid does not project from the longitudinal end of the folded part and does not have any influence on the surface of the outer layer, in addition to the effect of the invention of the first aspect.

Also in the catheter of the second aspect, the tip end of the braid is fixed in a series of operation by a so-called braider, which exerts the effect of facilitating fixing of the tip end of the braid.

The catheter according to the third aspect is the catheter according to the first aspect or the second aspect, further including a distal end tip connected to the distal end of the catheter main body, in which the inner layer and the braid extend to the distal end tip. Thus, the braid can be fixed to the inner layer without requiring a fixing tool for fixing the tip end of the braid.

Also in the catheter according to the third aspect, the tip end of the braid is fixed in a series of operation by a so-called braider, which exerts the effect of facilitating fixing of the tip end of the braid.

The catheter of the fourth aspect is any one of the catheters according to the first aspect to the third aspect, in which the braid is formed of a metal material. Thus, it is possible that the folded part of the braid functions as a marker for visually recognizing a position of the catheter, in addition to the effect of any one of the catheters according to the first aspect to the third aspect. Therefore, it is not necessary to provide a separate marker in the conventional manner.

Furthermore, in the catheter of the fourth aspect, the tip end of the braid is fixed and the marker is formed in a series of operation by a so-called braider, which exerts the effect of facilitating fixing of the tip end of the braid and formation of the marker.

### Brief Description of Drawings

FIG. 1 is a schematic side view of a catheter according to a first embodiment of the present invention.
FIG. 2 is an explanatory diagram for explaining a structure of a distal end of the catheter according to the first embodiment.
FIG. 3 is a longitudinal section view of the distal end of the catheter according to the first embodiment.
FIG. 4 is a longitudinal section view of a distal end of a catheter according to a second embodiment.
FIG. 5 is a schematic side view of a catheter according to a third embodiment.
FIG. 6 is a longitudinal section view of a distal end of the catheter according to the third embodiment. Description of Embodiments

The following will describe embodiments of the above-described present invention with reference to the enclosed drawings.

### (First embodiment)

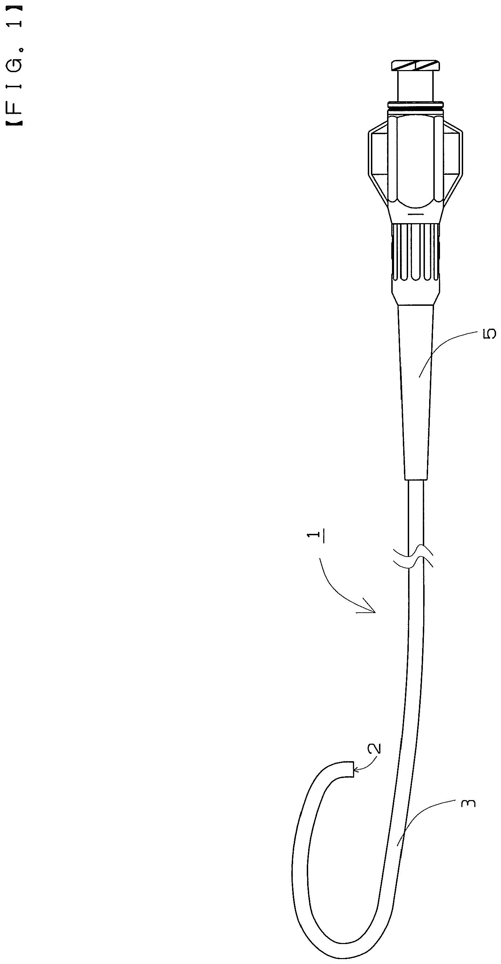

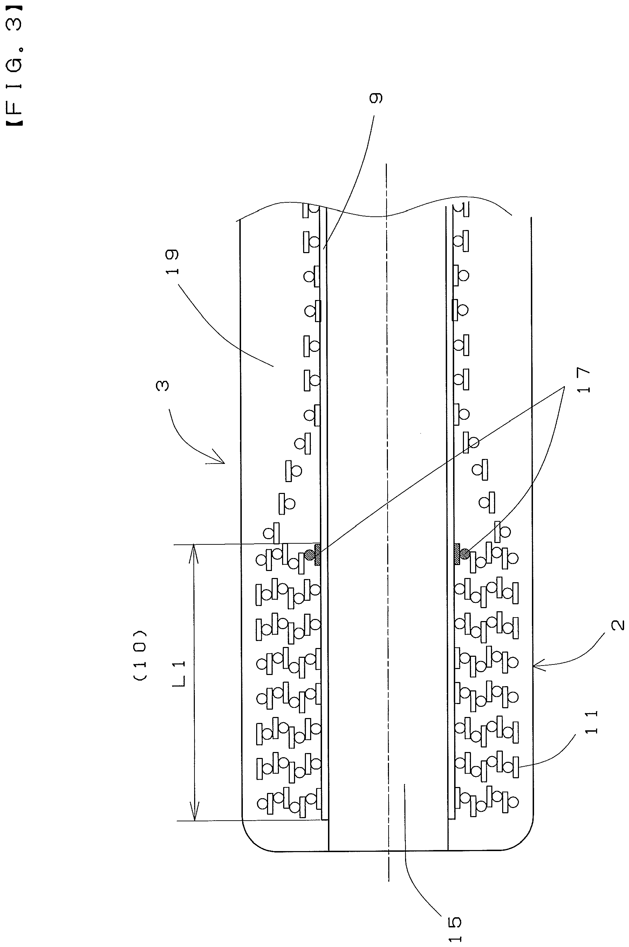

FIG. 1 is a schematic side view of a catheter according to a first embodiment of the present invention. FIG. 2 is an explanatory diagram for explaining a structure of a distal end of the catheter according to the first embodiment. FIG. 3 is a section view of the distal end of the catheter according to the first embodiment.

In FIG. 1 to FIG. 3, the left side of the drawings is a distal end side (far side) to be inserted in a body, while the right side is a proximal end side (near side) to be operated by a technician such as a physician. Note that each drawing is illustrated exaggeratedly for easier understanding, and the size is different from an actual size.

In FIG. 1, a catheter 1 is a tubular medical instrument with an entire length of 1200 mm. The catheter 1 includes a flexible catheter main body 3 and a connector 5 connected to a proximal end of the catheter main body 3.

As illustrated in FIG. 2, the catheter main body 3 has a three-layer structure including an inner layer 9 positioned on the inner side in a radial direction, a tubular braid (a tubular mesh) 11 covering an outer periphery of the inner layer 9, and a tubular outer layer 19 covering an outer periphery of the braid 11.

The inner layer 9 is a long hollow tubular body formed of resin, and has therein a lumen 15 for inserting a guide wire or other catheters. The resin material forming the inner layer 9 is not particularly limited. In the embodiment, polytetrafluoroethylene (PTFE) is used.

The braid 11 is formed of 16 (8 X 8) wires in total. More specifically, the braid 11 is formed of 8 first element wires 26a and 8 second element wires 26b that are alternately braided. In the embodiment, 8 first element wires 26a are wound in a counterclockwise direction toward the distal end, and 8 second element wires 26b are wound in a clockwise direction toward the distal end, as illustrated in FIG. 2.

The combination of element wires of the braid 11 is not limited to 8 wires x 8 wires in the embodiment. For example, the combination may be symmetrical with 4 wires x 4 wires, 2 wires x 2 wires or the like, or asymmetrical with 4 wires x 8 wires, 2 wires x 4 wires or the like.

Moreover, an element wire width of the first element wire 26a and an element wire width of the second element wire 26b may be same, or one of the element wire widths may be larger than the other.

The braid 11 of the embodiment is formed by alternately braiding the first element wires 26a and the second element wires 26b two by two. However, the embodiment is not limited thereto, and the braid 11 may be formed by alternately braiding them one by one.

The first element wires 26a and the second element wires 26b of the embodiment are formed of stainless steel (SUS304 or SUS316). However, there may be used metal other than stainless steel (e.g., platinum, tungsten, and the like) or a material other than metal (e.g., reinforced plastic).

Moreover, the material of the first element wires 26a and the second element wires 26b may be same or different.

When the first element wires 26a and/or the second element wires 26b are formed of metal element wires, they can function as a marker for visually recognizing a position of the catheter, as described later. Thus, it is not necessary to provide a separate marker.

In the embodiment, the first element wires 26a are round wires with a round section and the second element wires 26b are so-called flat wires with a rectangular section. However, there may have other sectional shapes, e.g., the first element wires 26a and the second element wires 26b may be round wires with a round section or flat wires with a rectangular section.

The braid 11 covers the outer periphery of the inner layer 9 from the distal end portion 2 to the proximal end portion of the inner layer 9, while it is folded back along the outer periphery of the inner layer 9 at the distal end portion 2 to form a multiple folded part 10 with a length L1, as illustrated in FIG. 2 and FIG. 3.

Moreover, a tip end 17 of the braid 11 of the embodiment is in contact with the inner layer 9, and is arranged in the lowest layer at the proximal end of the folded part 10.

The number of times of folding of the braid 11 in the present embodiment is not particularly limited as long as it is an even number. In the embodiment, the number of times of folding is four.

Regarding the order in which the folded part 10 is formed, the first element wires 26a and the second element wires 26b are first brought in contact with the inner layer 9. Then, the first (lowest) layer is braided from the tip end 17 of the braid 11 to be formed toward the distal end of the inner layer 9, and then the first element wires 26a and the second element wires 26b are folded back at the distal end portion of the folded part 10 to be formed to braid the second layer toward the proximal end of the inner layer 9. Thereafter, the first element wires 26a and the second element wires 26 are folded back at the proximal end portion of the folded part 10 to be formed to braid the third layer toward the distal end of the inner layer 9. Finally, the first element wires 26a and the second element wires 26 are folded back at the distal end portion of the folded part 10 to be formed to braid the fourth layer toward the proximal end of the inner layer 9, and the formation of the folded part 10 is finished. Subsequently, the first element wires 26a and the second element wires 26 are braided toward the proximal end of the inner layer 9 to form the braid 11 covering the inner layer 9.

Note that the tip end 17 of the braid 11 of the embodiment is positioned at the proximal end of the folded part 10. However, the tip end 17 may be positioned at the distal end of the folded part 10. In this case, the number of times of folding is an odd number (e.g., three times).

The outer layer 19 is formed of resin, and covers the inner layer 9 and the braid 11. The resin material forming the outer layer 19 is not particularly limited, and polyamide, polyamide elastomer, polyester, polyurethane, and the like can be used. In the embodiment, polyamide is used.

At the proximal end portion of the catheter main body 3, the proximal end portion of the inner layer 9, the proximal end portion of the braid 11, and the proximal end portion of the outer layer 19 are firmly fixed by the connector 5.

The catheter 1 of the embodiment includes the catheter main body 3 having the inner layer 9 that is a long hollow tubular body, the tubular braid 11 that covers the outer periphery of the inner layer 9 from the distal end to the proximal end of the inner layer 9, and the outer layer 19 covering the outer periphery of the braid 11. In the catheter 1 of the embodiment, the braid 11 is folded back at the distal end 2 of the inner layer 9, and the tip end 17 of the braid 11 is in contact with the inner layer 9 and is arranged in the lowest layer of the folded part 10. Thus, the braid 11 can be fixed to the inner layer 9 without requiring a fixing tool for fixing the tip end 17 of the braid 11 to the inner layer 9.

In the catheter 1 of the embodiment, the tip end 17 of the braid 11 is fixed in a series of operation by a so-called braider, which exerts the effect of facilitating fixing of the tip end 17 of the braid 11.

Moreover, even when the braid 11 of the catheter 1 of the embodiment is formed of low-cost stainless steel (SUS304 or SUS316), the folded part 10 can function as a marker for visually recognizing a position of the catheter. Therefore, it is not necessary to provide a separate marker in the conventional manner.

Furthermore, even when the braid 11 of the catheter 1 of the embodiment is formed of low-cost stainless steel (SUS304 or SUS316), the marker is formed in a series of operation by a so-called braider, which exerts the effect of facilitating formation of the marker.

The applicants formed the braid 11 using stainless steel (SUS304 and SUS316) and checked the visibility while setting the number of times of folding to two to four. It was confirmed that the visibility is improved when the number of times of folding is three times than when it is twice, and when it is four times than when it is three times. However, even when the number of times of folding is twice, the position of the catheter can be recognized visually, and it was confirmed that the folded part 10 can function as a marker.

In the embodiment, the folded part 10 of the braid 11 is provided at the distal end portion 2 of the inner layer 9. However, the folded part 10 may be provided at the proximal end portion of the inner layer 9. Also in such a case, the tip end 17 of the braid 11 may be provided at the distal end or the proximal end of the folded part 10.

### (Second embodiment)

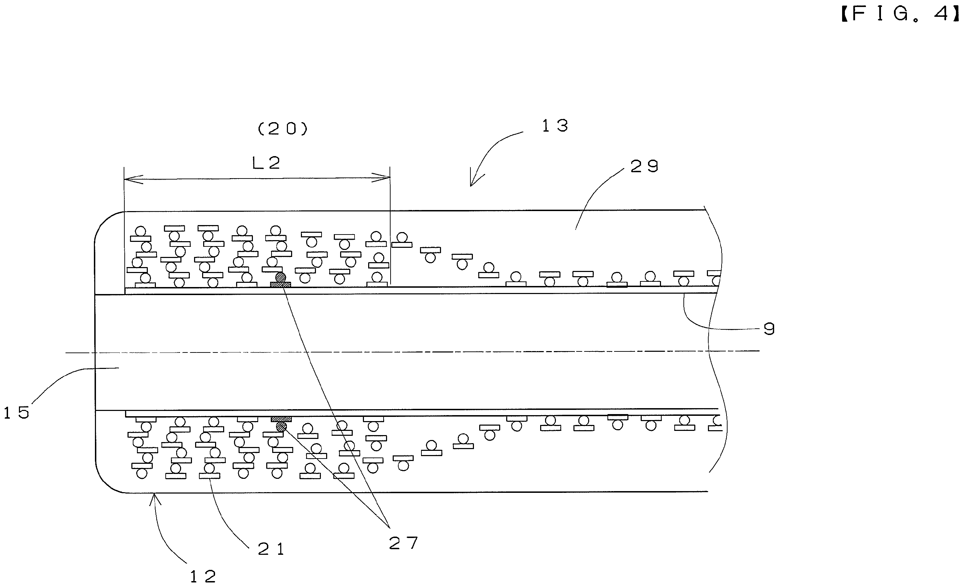

The following will describe the second embodiment of the present invention. The parts same as in the first embodiment are represented with same symbols in the drawing, and the explanation thereof is omitted. FIG. 4 is a section view of a distal end of a catheter according to the second embodiment.

In FIG. 4, the left side of the drawing is a distal end side (far side) to be inserted in a body, while the right side is a proximal end side (near side) to be operated by a technician such as a physician. Note that FIG. 4 is illustrated exaggeratedly for easier understanding, and the size is different from an actual size.

In FIG. 4, a catheter main body 13 has a three-layer structure including the inner layer 9 positioned on the inner side in a radial direction, a tubular braid (a tubular mesh) 21 covering a tubular outer periphery of the inner layer 9, and an outer layer 29 covering an outer periphery of the braid 21.

The braid 21 covers the outer periphery of the inner layer 9 from the distal end portion 12 to the proximal end portion of the inner layer 9, while it is folded back along the outer periphery of the inner layer 9 and braided at the distal end portion 12 to form a multiple folded part 20 with a length L2, as illustrated in FIG. 4.

Moreover, a tip end 27 of the braid 21 of the embodiment is in contact with the inner layer 9, and is arranged in the lowest layer in a middle position of the folded part 20.

Similarly to the braid 11 of the first embodiment, the braid 21 is formed of 16 (8 X 8) wires in total. More specifically, the braid 21 is formed of 8 first element wires 26a and 8 second element wires 26b that are alternately braided. The 8 first element wires 26a are wound in a counterclockwise direction toward the distal end, and the 8 second element wires are wound in a clockwise direction toward the distal end.

The combination of element wires of the braid 21 is not limited to 8 wires x 8 wires in the embodiment. For example, the combination may be symmetrical with 4 wires x 4 wires, 2 wires x 2 wires or the like, or asymmetrical with 4 wires x 8 wires, 2 wires x 4 wires or the like.

Moreover, an element wire width of the first element wires 26a and an element wire width of the second element wires 26b may be same, or one of the element wire widths may be larger than the other.

The braid 21 of the embodiment is formed by alternately braiding the first element wires 26a and the second element wires 26b two by two. However, the embodiment is not limited thereto, and the braid 21 may be formed by alternately braiding them one by one.

The first element wires 26a and the second element wires 26b of the embodiment are formed of stainless steel (SUS304 or SUS316). However, there may be used metal other than stainless steel (e.g., platinum, tungsten, and the like) or a material other than metal (e.g., reinforced plastic).

Moreover, the material of the first element wires 26a and the second element wires 26b may be same or different.

When the first element wires 26a and/or the second element wires 26b are formed of metal element wires, they can function as a marker for visually recognizing a position of the catheter. Thus, it is not necessary to provide a separate marker.

In the embodiment, there are used the first element wires 26a that are round wires with a round section and the second element wires 26b that are so-called flat wires with a rectangular section. However, they may have other sectional shapes, e.g., the first element wires 26a and the second element wires 26b may be round wire with a round section or flat wire with a rectangular section.

The number of times of folding of the braid 21 in the present embodiment is not particularly limited as long as it is an even number. In the embodiment, the number of times of folding is four.

Regarding the order in which the folded part 20 is formed, the first element wires 26a and the second element wires 26b are first brought in contact with the inner layer 9. Then, the first (lowest) layer is braided from the tip end 27 of the braid 21 to be formed toward the distal end of the inner layer 9, and then the first element wires 26a and the second element wires 26b are folded back at the distal end portion of the folded part 20 to be formed to braid the second layer toward the proximal end of the inner layer 9. Thereafter, the first element wires 26a and the second element wires 26b are folded back at the proximal end portion of the folded part 20 to be formed to braid the third layer toward the distal end of the inner layer 9. Finally, the first element wires 26a and the second element wires 26b are folded back at the distal end portion of the folded part 20 to be formed to braid the fourth layer toward the proximal end of the inner layer 9, and the formation of the folded part 20 is finished. Subsequently, the first element wires 26a and the second element wires 26b are braided toward the proximal end of the inner layer 9 to form braid 21 covering the inner layer 9.

Note that the first element wires 26a and the second element wires 26b are braided from the tip end 27 toward the distal end. However, they may be braided from the tip end 27 toward the proximal end. In this case, the number of times of folding is an odd number (e.g., three times).

The outer layer 29 is formed of resin, and covers the inner layer 9 and the braid 21. The resin material forming the outer layer 29 is not particularly limited, and polyamide, polyamide elastomer, polyester, polyurethane, and the like can be used. In the embodiment, polyurethane is used.

In the catheter of the embodiment, the tip end 27 of the braid 21 is in a middle position of the folded part 20. Thus, the tip end 27 of the braid 21 does not project from the longitudinal ends of the folded part 20 and does not have any influence on the surface of the outer layer 29.

Also in the catheter of the embodiment, the tip end 27 of the braid 21 is fixed in a series of operation by a so-called braider, which exerts the effect of facilitating fixing of the tip end 27 of the braid 21.

Moreover, even when the braid 21 of the catheter of the embodiment is formed of low-cost stainless steel (SUS304 or SUS316), the folded part 20 can function as a marker for visually recognizing a position of the catheter. Therefore, it is not necessary to provide a separate marker in the conventional manner.

Furthermore, even when the braid 21 of the catheter of the embodiment is formed of low-cost stainless steel (SUS304 or SUS316), the marker is formed in a series of operation by a so-called braider, which exerts the effect of facilitating formation of the marker.

Note that the tip end 27 of the braid 21 is arranged in a middle position of the folded part 20. However, the embodiment is not limited thereto, and the same effects can be obtained as long as the tip end 27 of the braid 21 is arranged in an area between both longitudinal ends of the folded part 20.

### (Third embodiment)

The following will describe the third embodiment of the present invention. The parts same as in the first embodiment are represented with same symbols in the drawings, and the explanation thereof is omitted.

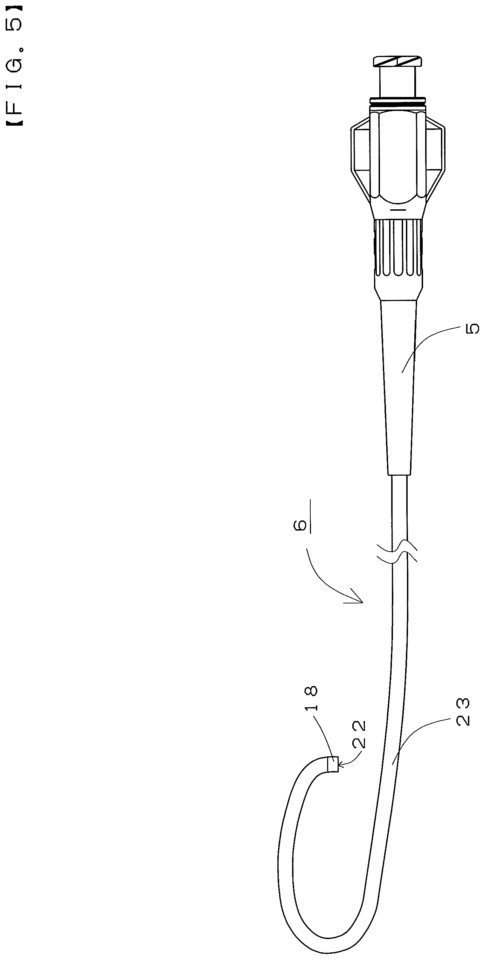

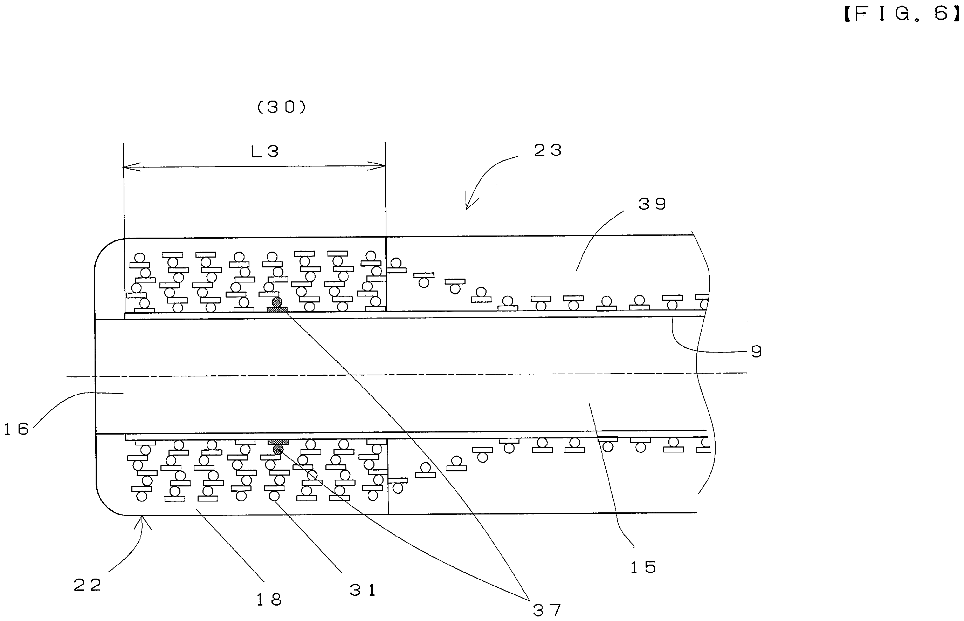

FIG. 5 is a schematic side view of a catheter according to a third embodiment of the present invention. FIG. 6 is a section view of a distal end of the catheter according to the third embodiment.

Also in FIG. 5 and FIG. 6, the left side of the drawings is a distal end side (far side) to be inserted in a body, while the right side is a proximal end side (near side) to be operated by a technician such as a physician. Note that each drawing is illustrated exaggeratedly for easier understanding, and the size is different from an actual size.

In FIG. 5, a catheter 6 is a tubular medical instrument with an entire length of 1200 mm. The catheter 6 includes a flexible catheter main body 23, a distal end tip 18 connected to the distal end of the catheter main body 23, and the connector 5 connected to the proximal end of the catheter main body 23.

The distal end tip 18 is a cylindrical member having an opening 16 communicating with the lumen 15. The resin forming the distal end tip 18 is not particularly limited, and polyurethane, polyurethane elastomer, and the like are used.

The distal end tip 18 may contain radiopaque powder. For example, when the distal end tip 18 contains radiopaque powder (e.g., tungsten powder) in a range of about 65w% to about 90w%, a technician such as a physician can accurately grasp a position of the catheter in coronary angiography.

The catheter main body 23 has a three-layer structure including the inner layer 9 positioned on the inner side in a radial direction, a tubular braid (a tubular mesh) 31 covering an outer periphery of the inner layer 9, and a tubular outer layer 39 covering an outer periphery of the braid 31, as illustrated in FIG. 6.

The braid 31 covers the outer periphery of the inner layer 9 from the distal end portion 22 to the proximal end portion of the inner layer 9, while it is folded back along the outer periphery of the inner layer 9 and braided at the distal end portion 22 to form a multiple folded portion 30 with a length L3, as illustrated in FIG. 6.

Moreover, a tip end 37 of the braid 31 of the embodiment is in contact with the inner layer 9, and is arranged in the lowest layer in a middle position of the folded part 30.

Similarly to the braid 11 of the first embodiment, the braid 31 is formed of 16 (8 X 8) wire in total. More specifically, the braid 31 is formed of 8 first element wires 26a and 8 second element wires 26b that are alternately braided. The 8 first element wires 26a are wound in a counterclockwise direction toward the distal end, and the 8 second element wires 26b are wound in a clockwise direction toward the distal end.

The combination of element wires of the braid 31 is not limited to 8 wires x 8 wires in the embodiment. For example, the combination may be symmetrical with 4 wires x 4 wires, 2 wires x 2 wires or the like, or asymmetrical with 4 wires x 8 wires, 2 wires x 4 wires or the like.

Moreover, an element wire width of the first element wires 26a and an element wire width of the second element wires 26b may be same, or one of the element wire widths may be larger than the other.

The braid 31 of the embodiment is formed by alternately braiding the first element wires 26a and the second element wires 26b two by two. However, the embodiment is not limited thereto, and the braid 31 may be formed by alternately braiding them one by one.

The first element wires 26a and the second element wires 26b of the embodiment are formed of stainless steel (SUS304 or SUS316). However, metal other than stainless steel (e.g., platinum, tungsten, and the like) or a material other than metal (e.g., reinforced plastic) may be used.

Moreover, the material of the first element wires 26a and the second element wires 26b may be same or different.

When the first element wires 26a and/or the second element wires 26b are formed of metal element wires, they can function as a marker for visually recognizing a position of the catheter. Thus, it is not necessary to provide a separate marker.

In the embodiment, the first element wires 26a are round wires with a round section and the second element wires 26b are so-called flat wire with a rectangular section. However, they may have other sectional shapes, e.g., the first element wires 26a and the second element wires 26b may be round wires with a round section or flat wires with a rectangular section.

The number of times of folding of the braid 31 in the present embodiment is not particularly limited as long as it is an even number. In the embodiment, the number of times of folding is four.

Regarding the order in which the folded part 30 is formed, the first element wires 26a and the second element wires 26b are first brought in contact with the inner layer 9. Then, the first (lowest) layer is braided from the tip end 37 of the braid 31 to be formed toward the distal end of the inner layer 9, and then the first element wires 26a and the second element wires 26b are folded back at the distal end portion of the folded part 30 to be formed to braid the second layer toward the proximal end of the inner layer 9. Thereafter, the first element wires 26a and the second element wires 26b are folded back at the proximal end portion of the folded part 30 to be formed to braid the third layer toward the distal end of the inner layer 9. Finally, the first element wires 26a and the second element wires 26b are folded back at the distal end portion of the folded part 30 to braid the fourth layer toward the proximal end of the inner layer 9, and the formation of the folded part 30 is finished. Subsequently, the first element wires 26a and the second element wires 26b are braided toward the proximal end of the inner layer 9 to form braid 31 covering the inner layer 9.

Note that the first element wires 26a and the second element wires 26b are braided from the tip end 37 toward the distal end of the inner layer 9. However, they may be braided from the tip end 37 toward the proximal end of the inner layer 9. In this case, the number of times of folding is an odd number (e.g., three times).

The outer layer 39 is formed of resin, and covers the inner layer 9 and the braid 31. The resin material forming the outer layer 39 is not particularly limited, and polyamide, polyamide elastomer, polyester, polyurethane, and the like can be used. In the embodiment, polyurethane is used.

The catheter 6 of the embodiment includes the distal end tip 18 connected to the distal end of the catheter main body 23, and the inner layer 9 and the braid 31 extend to the distal end tip 18. The distal end tip 18 includes the folded part of the braid 31. Thus, the braid 31 can be fixed to the inner layer 9 without requiring a fixing tool for fixing the tip end 37 of the braid 31.

Also in the catheter 6 of the embodiment, the tip end 37 of the braid 31 is fixed in a series of operation by a so-called braider, which exerts the effect of facilitating fixing of the tip end 37 of the braid 31.

In the catheter 6 of the embodiment, the tip end 37 of the braid 31 is arranged in a middle position of the folded part 30. Thus, the tip end 37 of the braid 31 does not project from the longitudinal ends of the folded part 30 and does not have any influence on the surface of the outer layer 39.

Moreover, even when the braid 31 of the catheter 6 of the embodiment is formed of low-cost stainless steel (SUS304 or SUS316), the folded part 30 can function as a marker for visually recognizing a position of the catheter. Therefore, it is not necessary to provide a separate marker in the conventional manner.

Furthermore, even when the braid 31 of the catheter 6 of the embodiment is formed of low-cost stainless steel (SUS304 or SUS316), the marker is formed in a series of operation by a so-called braider, which exerts the effect of facilitating formation of the marker.

Note that in the embodiment, the tip end 37 of the braid 31 is arranged in a middle position of the folded part 30. However, the embodiment is not limited thereto, and the same effect can be obtained as long as the tip end 37 of the braid 31 is arranged in an area between both longitudinal ends of the folded part 30.

At the proximal end portion of the catheter main body 23, the proximal end portion of the inner layer 9, the proximal end portion of the braid 31, and the proximal end portion of the outer layer 39 are firmly fixed by the connector 5.

### Reference Signs List

- 1, 6: catheter

- 9: inner layer
- 10, 20, 30: folded part
- 11, 21, 31: braid
- 15: lumen
- 17, 27, 37: braid end
- 18: distal end tip
- 19, 29, 39: outer layer
- 26a: first element wire
- 26b: second element wire

## Claims

1. A catheter (1, 6) comprising:
an inner layer (9) that is a long hollow tubular body,
a tubular braid (11, 21, 31) that covers an outer periphery of the inner layer (9), and
a tubular outer layer (19, 29, 39) that covers an outer periphery of the braid (11, 21, 31), wherein
the braid (11, 21, 31) is folded back at least at one of the longitudinal ends of the inner layer (9) to form a multiple folded part (10, 20, 30) so that a tip end (17, 27, 37) of the braid (11, 21, 31) is in contact with the inner layer (9) and is arranged in a lowest layer of the folded part (10, 20, 30).

2. The catheter according to claim 1, wherein the tip end (27, 37) of the braid (21, 31) is positioned in an area between the two longitudinal ends of the folded part (20, 30).

3. The catheter according to claim 1 or 2, further comprising a catheter main body (3, 13) including the inner layer (9), the braid (11, 21, 31) and the outer layer (19, 29, 39).

4. The catheter according to claim 3, further comprising a distal end tip (18) connected to a distal end of the catheter main body (23), wherein
the inner layer (9) and the braid (31) extend to the distal end tip (18).

5. The catheter according to claim 4, wherein the distal end tip (18) includes the folded part (30).

6. The catheter according to any one of claims 1 to 5, wherein the braid (11, 21, 31) is formed of a metal material.

## Patentansprüche

1. Katheter (1, 6) mit:
einer Innenschicht (9), die ein langer rohrförmiger Hohlkörper ist,
einem rohrförmigen Geflecht (11, 21, 31), das den Außenumfang der Innenschicht (9) ummantelt, und
einer rohrförmigen Außenschicht (19, 29, 39), die den Außenumfang des Geflechts (11, 21, 31) ummantelt, wobei
das Geflecht (11, 21, 31) wenigstens an einem der Längenenden der Innenschicht (9) zurückgeschlagen ist, um einen mehrfach umgeschlagenen Teil (10, 20, 30) dergestalt zu bilden, dass das Spitzenende (17, 27, 37) des Geflechts (11, 21, 31) mit der Innenschicht (9) in Kontakt ist und in der innersten Schicht des umgeschlagenen Teils (10, 20, 30) liegt.

2. Katheter nach Anspruch 1, wobei das Spitzenende (27, 37) des Geflechts (21, 31) in einem Bereich zwischen den beiden Längenenden des umgeschlagenen Teils (20, 30) liegt.

3. Katheter nach Anspruch 1 oder 2, des Weiteren mit einem Katheterhauptkörper (3, 13), der die Innenschicht (9), das Geflecht (11, 21, 31) und die Außenschicht (19, 29, 39) aufweist.

4. Katheter nach Anspruch 3, des Weiteren mit einer distalen Endspitze (18), die mit einem distalen Ende des Katheterhauptkörpers (23) verbunden ist, wobei
die Innenschicht (9) und das Geflecht (31) sich zur distalen Endspitze (18) hin erstrecken.

5. Katheter nach Anspruch 4, wobei die distale Endspitze (18) den umgeschlagenen Teil (30) aufweist.

6. Katheter nach einem der Ansprüche 1 bis 5, wobei das Geflecht (11, 21, 31) aus einem Metallmaterial gebildet ist.

## Revendications

1. Cathéter (1, 6) comprenant :
une couche interne (9) qui est un corps tubulaire creux long,
une tresse tubulaire (11, 21, 31) qui recouvre une périphérie externe de la couche interne (9), et
une couche externe tubulaire (19, 29, 39) qui recouvre une périphérie externe de la tresse (11, 21, 31), dans lequel :
la tresse (11, 21, 31) est repliée au moins au niveau d'une extrémité des extrémités longitudinales de la couche interne (9) afin de former une partie pliée plusieurs fois (10, 20, 30) de sorte qu'une extrémité de pointe (17, 27, 37) de la tresse (11, 21, 31) est en contact avec la couche interne (9) et est agencée dans la couche la plus basse de la partie pliée (10, 20, 30).

2. Cathéter selon la revendication 1, dans lequel l'extrémité de pointe (27, 37) de la tresse (21, 31) est positionnée dans une zone entre deux extrémités longitudinales de la partie pliée (20, 30).

3. Cathéter selon la revendication 1 ou 2, comprenant en outre un corps principal de cathéter (3, 13) comprenant la couche interne (9), la tresse (11, 21, 31) et la couche externe (19, 29, 39).

4. Cathéter selon la revendication 3, comprenant en outre une pointe d'extrémité distale (18) raccordée à une extrémité distale du corps principal de cathéter (23), dans lequel :
la couche interne (9) et la tresse (31) s'étendent vers la pointe d'extrémité distale (18).

5. Cathéter selon la revendication 4, dans lequel la pointe d'extrémité distale (18) comprend la partie pliée (30).

6. Cathéter selon l'une quelconque des revendications 1 à 5, dans lequel la tresse (11, 21, 31) est formée à partir d'un matériau métallique.
